Europäisches Patentamt

(19) **European Patent Office**

**Office européen des brevets**

(11) Publication number: **0 078 621**
**A1**

(12) **EUROPEAN PATENT APPLICATION**

(21) Application number: **82305504.1**

(51) Int. Cl.³: **C 12 P 19/06**

(22) Date of filing: **15.10.82**

---

(30) Priority: **29.10.81 GB 8132564**

(43) Date of publication of application: **11.05.83**
**Bulletin 83/19**

(84) Designated Contracting States: **DE FR NL**

(71) Applicant: **Kelco Biospecialties Limited, 22 Henrietta Street, London WC2E 8NB (GB)**

(72) Inventor: **Smith, Ian Humphrey, 58 Fitzroy Crescent Woodley, Reading Berkshire (GB)**
Inventor: **Symes, Kenneth Charles, 24/26 Queen's Road, Reading Berkshire (GB)**

(74) Representative: **Ablewhite, Alan James et al, MARKS & CLERK 57/60 Lincoln's Inn Fields, London WC2A 3LS (GB)**

---

(54) **Treatment of Xanthan gum solutions.**

(57) A process for improving the clarity and filterability of xanthan gum solution comprises:
   a) treating a crude xanthan gum solution at a pH of 2 to 7 with an effective amount of an acid or neutral protease, and then
   b) raising the pH to 8–13 and, optionally,
   c) then lowering the pH to 6–7.

EP 0 078 621 A1

1.

K-2057A

## "TREATMENT OF XANTHAN GUM SOLUTION"

The present invention relates to the treatment of solutions of xanthan gum, and in particular to the use of enzymes to treat such solutions in order to impart clarity and in order to improve filterability.

The presence of non-viable microbial cells in crude xanthan gum solutions such as xanthan gum fermentation broths and crude xanthan gum preparations isolated from whole broths can result in adverse effects when the preparations are to be used in applications such as food formulation or enhanced oil recovery.

UK Patent Specification No. 1443507 teaches that a certain type of enzyme, the bacterial alkaline proteases, can be used to degrade bacterial cells of the polysaccharide producers of the genus Xanthamonas. The references cited in the specification indicate that the alkaline proteases which are to be used are characterized by their high proteolytic activity above pH 7, by the presence of an esterase activity and by the presence of a serine moiety at the active site which is inactivated by diisopropyl fluorophosphate. In general, the preferred treatment in accordance

2.

0078621

with this UK Patent involves holding a solution of the gum and alkaline protease at pH 7 to 12 for 20 - 25 hours at from room temperature to 60°C.

Working with the bacterial alkaline protease sold as Esperase (Registered Trade Mark), O.M. Stokke has described in US Patent No. 4,165,257 a modified procedure for the treatment of xanthan gum and other polymers in which the polymer is treated with a specific Bacillus enzyme at an exceptionally high pH, namely a pH between 12.5 and 13.0. By simultaneously using the alkaline protease and the high pH, it is said that a synergistic effect is obtained.

We have discovered that it is not necessary to use alkaline proteases as the enzyme to treat xanthan gum solutions, and that treatment with base on its own following a treatment with other enzymes can lead to effective clarification and good filterability of xanthan gum.

In accordance with the present invention there is provided a method for improving the clarity and filterability of a xanthan gum solution containing bacterial cells in which method firstly the solution is treated with an acid or neutral protease and secondly the solution is treated with an alkali.

It is essential that the treatment steps are performed in the specified sequence.

3. 0078621

The enzymes employed in the present method include acid proteases from bacterial, fungal or plant sources, as described for example in A. Yamamoto, Proteolytic Enzymes in "Enzymes in Food Processing", Ed.G.Reed. 1975, Academic Press.

Whereas the processes known from UK Patent Specification No. 1443507 and US Patent No. 4,165,257 employ enzymes with a serine moiety at the active site, the present method employs enzymes which have a different functionality at the active site. This difference in functionality is in turn reflected by the different proteolytic characteristics which are used to advantage in the present method.

The acid proteases are normally most active in the pH range of 2 to 6, and are insensitive to certain reagents which inhibit alkaline proteases, while the neutral proteases are normally most active at a pH of 6 to 7. In contrast, the alkaline proteases exhibit maximum activity at a pH of 9.5 to 10.5.

Proteases for use in the present method are readily available. Such proteases include for example the acid proteases elaborated by Aspergillus niger or present in pineapples.

In using the acid proteases the pH of the solution being treated is suitably from 2 to 7, preferably from 3 to 6. For neutral proteases the pH range is suitably 6 to 7.

The level of protease employed will depend on the nature of the xanthan solution being treated and on the conditions to be employed in effecting the clarification. For instance, with whole broths of xanthan (the polysaccharide produced by _Xanthomonas spp_), a concentration of 0.2 to 4 gram of enzyme per litre of solution will usually be found to be satisfactory.

In performing the present method, the degradation of the bacterial cells is achieved by incubating the enzyme-containing solution for a suitable period, eg from 0.5 to 10 hours, and preferably at above room temperature. Preferred incubation conditions are 0.5 to 2, e.g. 1 hour, at $50^{\circ}$ to $70^{\circ}$, e.g. $60^{\circ}C$.

After the treatment with acid or neutral protease the solution has to be treated with alkali. This alkali treatment helps to improve still further the filterability of the xanthan gum solution, above and beyond the improvement afforded by the enzyme treatment.

Suitable alkalies include alkali metal hydroxides, especially sodium or potassium hydroxide; alkaline earth metal hydroxides, especially calcium hydroxide; and alkali metal carbonates, especially sodium carbonate. Preferably sufficient alkali is added to raise the pH of the xanthan gum solution beyond pH 8. In particular, the pH should be taken to from pH 8 to pH 13, with pH 11.5 to 12.5 being especially suitable.

Unlike other base treatments described in the literature for treatment of xanthan gum, we prefer not to employ an elevated temperature.

In US patent No.3,355,447 there is mention of adjustment of the pH to 8 to 9 and effecting a heat treatment at 150 to 170$^o$F (about 66 to 77$^o$C); in US Patents No.3,729,460 and No.3,964,972 there is claimed a method involving adjustment of the pH to above 8 and heating the reaction mixture to 150 to 250$^o$C (about 66$^o$ to about 121$^o$C); and in UK Patent Specification No.1,531,970 there is mention of treatment of a fermented xanthan gum broth with an alkali metal hydroxide at a temperature of 55$^o$ - 121$^o$C. None of these prior patents suggest that further improvement of the gum characteristics can be achieved by the use of the known treatment with alkaline proteases, let alone by the use of the acid or neutral proteases adopted by the present invention.

More particularly, a treatment with alkali in accordance with the present invention is best effected at 15 to 40$^o$C, eg 20 to 25$^o$C, for 0.5 to 10 hours, eg 0.5 to 2 hours.

After the alkali treatment it is preferred to adjust the pH to 7 or below by the addition of acid: in particular adjustment to pH 6 or 7 with sulphuric or hydrochloric acid is especially suitable.

The present invention is illustrated by the following nonlimiting Examples which describe processes in accordance with the present invention. The evaluation of the treated xanthan gum solutions is then given in comparison with some xanthan gum solutions treated by related procedures described in Comparison Examples.

Example 1

A 1 litre sample of a batch of freshly fermented broth containing about 2.5% by weight of xanthan gum was given two treatments in the following order:

(i) The broth was vigorously stirred using a Silverson mixer, thereby raising the temperature of the broth to 60°C. Sufficient hydrochloric acid was then added to adjust the pH to 5.5 and this was followed by the addition of 5g of an enzyme preparation known as Progan (Biocon (UK) Ltd) containing an acid protease (papain) and a β-glucanase. Mixing was maintained for a period of 1h whereupon the broth was neutralised with 10N sodium hydroxide solution and allowed to cool to below a temperature of 30°C.

(ii) The broth was then base treated at about 25°C with 10N sodium hydroxide such that the pH was raised above 11.5; extra base was added as necessary to maintain these pH conditions for 1h. Finally the broth was neutralised with 2N hydrochloric acid.

Comparative Example 1 (a)

For comparison, a further sample of the same xanthan broth as used in Example 1 was treated in the way specified in the first treatment in Example 1 but was not then given the second treatment employing base.

7. 0078621

## Comparative Example 1 (b)

For further comparison, a third sample of the same xanthan broth as used in Example 1 was treated in the way specified in Example 1 but with omission from the first treatment of the enzyme addition.

## Comparative Example 1 (c)

For further comparison, a fourth sample of the same xanthan broth as used in Example 1 was treated in the way specified in Example 1 but with omission of the enzyme addition from the first treatment and with omission of the second treatment employing base.

## Comparative Example 1 (d)

For further comparison, a fifth sample of the same xanthan broth as used in Example 1 was treated in the way specified in Example 1 but with omission of the first treatment.

## Comparative Example 1 (e)

For further comparison, a sixth sample of the same xanthan broth as used in Example 1 was treated in the way specified in Example 1 except that the second treatment employing base was carried out before the first treatment employing enzyme.

0078621

## Comparative Example 1 (f)

For yet further comparison, a seventh sample of the same xanthan broth as used in Example 1 was left untreated.

## Example 2

The procedure of Example 1 was repeated on a 1 l sample from a second batch of 0.1% (w/v) xanthan gum broth except that in the first treatment, the temperature was $50^{o}C$, the pH was adjusted to 6 with sulphuric acid and 0.8g of a neutral protease known as Neutrase (Novo Industri A/S) was added. The mixing period after enzyme addition was 2h. In the second treatment the final neutralisation was with 2$\underline{N}$ sulphuric acid.

## Comparative Example 2 (a)

For comparison, a further sample of the same xanthan broth as used in Example 2 was treated in the way specified in the first treatment in Example 2 but was not then given the second treatment employing base.

## Comparative Example 2 (b)

For further comparison a third sample of the same xanthan broth as used in Example 2 was untreated.

## Example 3

The procedure of Example 1 was repeated on a 1 l sample from a batch of 0.2% (w/v) xanthan gum broth except that in the first treatment the pH was adjusted to 6, the temperature was $37^{o}C$ and 5g of the enzyme known as Yeast Lytic Enzyme YL5 (Amano Pharmaceutical Co., Ltd) was added.

## Comparative Example 3 (a)

For comparison, a further sample of the same xanthan broth as used in Example 3 was treated in the way specified in the first treatment in Example 3 but was not then given the second treatment employing base.

## Comparative Example 3 (b)

For further comparison, a third sample of the same xanthan broth as used in Example 3 was untreated.

Assessment of Filterability of the Treated Xanthan Gum Solutions.

Theory

Koziki et al (Can. J.Chem.Eng 46, 313 (1968) have derived equations of motion characterising the flow of incompressible, time-independent non-Newtonian fluids, exhibiting a so-called anomalous surface effect, through compressible porous media. Approximations were made to arrive at workable constant pressure and constant flow rate filtration equations for slurries of non-Newtonian (power law) fluids. These equations were verified experimentally using slurries of calcium carbonates in water and dilute CMC solutions.

The general theoretical equation derived by Kozicki et al for power law fluids is as follows:

$$dt = \left(\frac{K_c \cdot \Upsilon}{\Delta P}\right)^{1/n} \left(1 - \frac{U_{ws}}{U_s}\right) \left(\frac{V}{A} + \frac{R_m}{c\Upsilon}\right) d\left(\frac{V}{A}\right) \quad \ldots\ldots(1)$$

where t = filtering time / s

K = consistency index / dyne $cm^{-2}$ $s^n$

n = flow behaviour index

c = mass of particles deposited per unit volume of filtrate / g $cm^{-3}$

$\gamma$ = average specific cake resistance for power law fluids / $cm^{2-n}$ $g^{-1}$

$\Delta P$ = filtering pressure / dynes $cm^{-2}$

$U_{ws}$ = slip velocity on flow path wall / cm $s^{-1}$

$U_s = d(V/A)/dt$ = mean flow velocity in flow path / cm $s^{-1}$

V = filtrate volume / $cm^3$

A = cross-sectional area of filter beds / $cm^2$

$R_m$ = filter medium resistance / $cm^{-n}$

By re-arranging this equation and adopting the procedure of Miura et al (J Ferment Technol 54, 667 (1976) ), equation (1) simplified to:

$$\frac{\Delta P}{K} \left[ \frac{d(V/A)}{dt} \right]^{-n} = c\gamma \left( \frac{V}{A} \right) + R_m$$

Thus a graph of $\Delta P/k[d(V/A)/dt]^{-n}$ versus (V/A) should be linear with a slope of $c\gamma$ and intercept $R_m$ . The smaller the gradient $(c\gamma)$ the better the filtration characteristics.

In this way it is possible to make direct comparisons of the performance of viscous solutions whilst compensating for the effects of different rheology on the filtration rate.

Practice

The treated broths from Examples 1 and 2 and Comparative Examples 1 (a) to 1 (f), 2 (a) and 2 (b) were evaluated at 0.1% w/v gum concentration after dilution with sodium chloride solution such that the final solution contained 3% NaCl. The treated broths from Example 3 and Comparative Examples 3 (a) and 3 (b) were evaluated at 0.2% w/v gum concentration after dilution with sodium chloride solution such that the final solution contained 3% NaCl.

The dilution on a w/v basis was made using the known polysaccharide content (total isopropanol precipitated matter in $gl^{-1}$), assuming that the broth density was unity. The diluted broth was stirred under low-shear conditions with a Citenco stirrer for 1 h and allowed to stand in a water bath at $25^{\circ}C$ for 10 - 15 minutes before being subjected to a standardized filtration test.

The apparatus for the filtration test comprised a cylindrical stainless steel Millipore pressure holder (340 ml) maintained at a constant temperature of 25$^{\circ}$C by circulating water from a thermostat bath through a surrounding coil of copper pipe. The holder was fitted with a 0.8 $\mu$m Millipore (47 mm dia) MF filter with a superposed Millipore AP20 (42 mm dia) disc prefilter. A standard volume of test solution (200 ml) at 25$^{\circ}$C was introduced and the unit pressurised with air at a constant level of 1050 mm Hg monitored with a Wallace and Tiernan pressure gauge. The cumulative mass throughput of solution was monitored as a function of time by collecting the filtrate in a beaker on an I.E. DSK 2 electronic balance with output to a chart recorder (Philips PM 8202) running at a suitable chart speed.

For treatment of the filtration results, the mass flow rate (dM/dt) was calculated as a function of the mass M of accumulated filtrate from measurements of the slope of tangents drawn to the traces of M versus time t generated by the chart recorder. The density of the filtrate was measured by weighing a known volume ( 10 ml) at 25$^{\circ}$C and then used to convert the filtrate mass to volume V. The area A of the filter was 11.3 cm$^2$. The power law parameters K and n were determined on a sample of the test solution at 25$^{\circ}$C from flow curves obtained using a Contraves Rheomat 30 viscometer with a twin-gap, double-concentric cylinder device (MS-0). The data were used to calculate $\frac{\Delta P}{K}[d(V/A)/dt]^{-n}$ as a function of (V/A) for each solution filtered.

14. 0078621

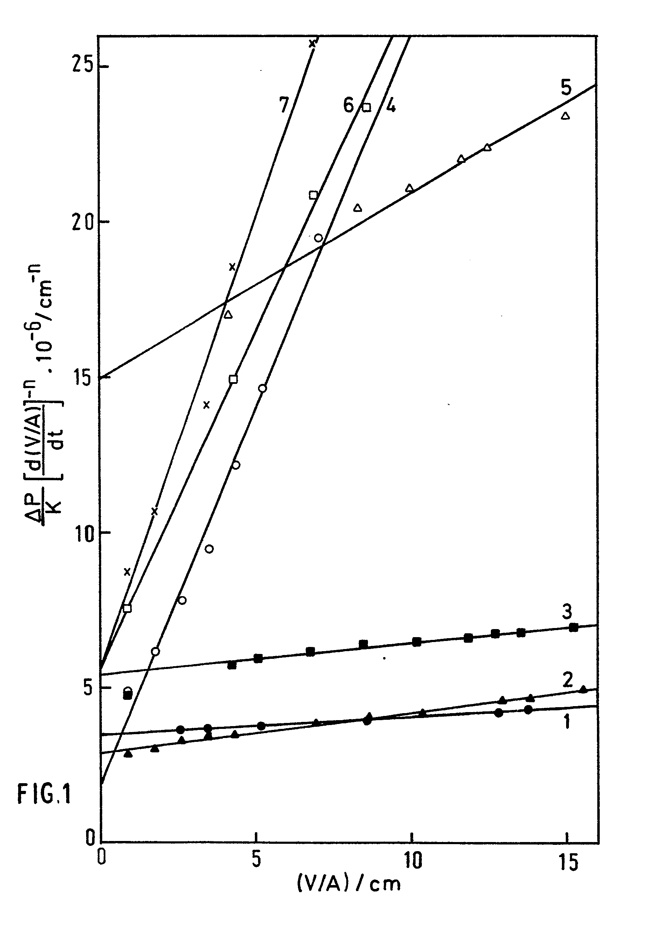

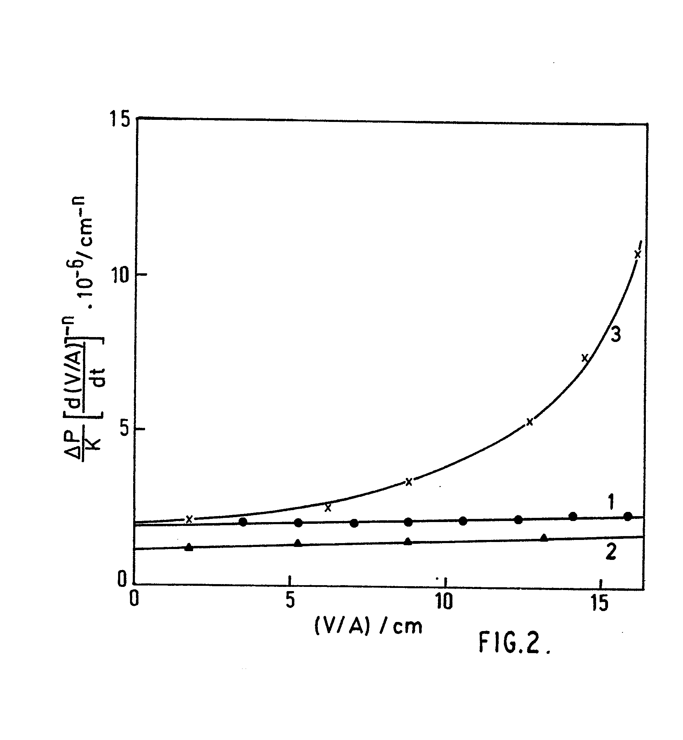

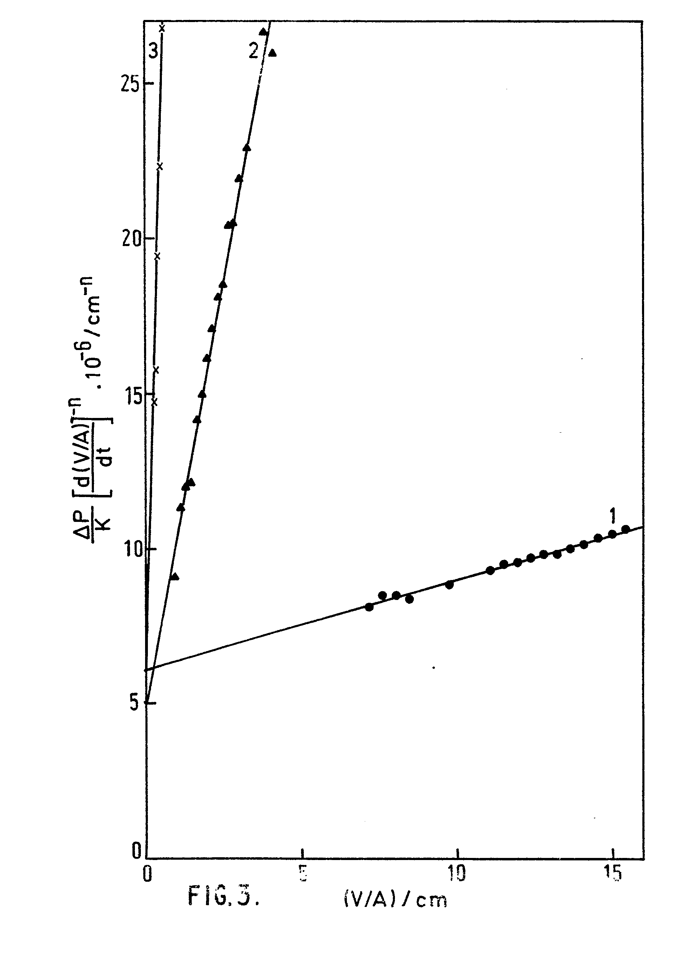

The results for all the examples were processed to provide the plots shown in Figures 1, 2 and 3.

Table 1 shows the correspondence between the numerically labelled plots in each Figure and the Example or Comparative Example number together with summaries of the broth treatments, the concentration of xanthan in the diluted broths tested and the gradients $c\gamma$ of the plots.

15.

Table 1

| Fig No. | Plot No. | Example No. | Comparative Example No. |
|---------|----------|-------------|-------------------------|
| 1 | 1 | 1 | - |
|   | 2 | - | 1 (a) |
|   | 3 | - | 1 (b) |
|   | 4 | - | 1 (c) |
|   | 5 | - | 1 (d) |
|   | 6 | - | 1 (e) |
|   | 7 | - | 1 (f) |
| 2 | 1 | 2 | - |
|   | 2 | - | 2 (a) |
|   | 3 | - | 2 (b) |
| 3 | 1 | 3 | - |
|   | 2 | - | 3 (a) |
|   | 3 | - | 3 (b) |

Table 1 cont..

| Summary of broth treatment | Xanthan conc in diluted broth % w/v | Gradient $10^4 . \; c \; /cm^{-(n+1)}$ |
|---|---|---|
| (i) stirred, 60°C, pH5.5, Progan 1h, neutralised, cooled<br>(ii) pH >11.5, 1h, neutralised | | 5.95 |
| (i) stirred, 60°C, pH5.5, Progan<br>(ii) nil | | 13.4 |
| (i) stirred, 60°C, pH5.5, 1h, neutralised, cooled<br>(ii) pH >11.5, 1h, neutralised | | 9.91 |
| (i) stirred, 60°C, pH5.5, 1h,<br>(ii) nil | 0.1 | 242 |
| (i) pH >11.5, 1h, neutralised<br>(ii) nil | | 59.2 |
| (i) pH >11.5, 1h, neutralised<br>(ii) stirred, 60°C, pH5.5, Progan 1h, neutralised, cooled | | 216 |
| Untreated | | 289 |
| (i) stirred, 50°C, pH6, Neutrase, 2h, neutralised, cooled<br>(ii) pH >11.5, 1h, neutralised | | 2.78 |
| (i) stirred 50°C, pH6, Neutrase, 2h, neutralised, cooled<br>(ii) nil | 0.1 | 3.55 |
| Untreated | | non linear plot |
| (i) stirred, 60°C, pH5.5, YL5, 1h, neutralised, cooled<br>(ii) pH >11.5, 1h, neutralised | | 29.5 |
| (i) stirred, 60°C, pH5.5, YL5, 1h, neutralised, cooled<br>(ii) nil | 0.2 | 552 |
| Untreated | | 3315 |

In each of the three figures it is seen that the diluted broth with the lowest gradient relative to that of the untreated broth (ie with the most improvement in filtration characteristics) was the one which had been given, prior to dilution, a first treatment employing enzyme and then a second treatment employing base (ie Examples 1, 2 and 3 gave the best results).

Comparative Examples 1(a), 2(a) and 3(a) illustrate that a significant but somewhat lesser improvement is obtained if the second treatment employing base is omitted. This finding also applies to Comparative Example 1(b) in which the enzyme addition was omitted from the first treatment. Comparative Example 1(d) shows that a moderate improvement can be made when the first treatment employing enzyme is omitted. Very poor effects were obtained in Comparative Example 1(c) where the enzyme addition from the first treatment and the second treatment employing base were omitted and in Comparative Example 1(e) where the second treatment was carried out before the first.

CLAIMS

1.    A process for improving the clarity and filterability of xanthan gum solution which comprises:

a)    treating a crude xanthan gum solution at a pH of 2 to 7 with an effective amount of an acid or neutral protease, and then

b)    raising the pH to 8 - 13

2.    A process of claim 1 wherein in step (a) the protease is an acid protease, the pH is 2 - 7, and the temperature is about 50-70°C  for 0.5 to 10 hours.

3.    A process of claim 1 wherein the protease is a neutral protease, the pH is 6 - 7, and the temperature is about 50-70°C for 0.5 to 10 hours.

4.    A process of claim 2 or 3 wherein the temperature is about 60°C for about 0.5 to 2 hours.

5.    A process of claim 1 wherein in step (b) the pH is about 11.5 - 12.5.

6.    A process of claim 1 wherein in step (b) the temperature is about 15-40°C for 0.5 to 10 hours.

7.    A process of claim 6 wherein the temperature is 20 - 25°C for 0.5 to 2 hours.

8.    A process of claim 1 further comprising:

(c)    after step (b), lowering the pH to 6 - 7.

FIG.1

0078621

FIG.2.

FIG.3.

# 0078621

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| | --- | | C 12 P 19/06 |
| X,Y | GB-A-2 065 689 (TATE & LYLE LTD.) <br> * Whole document * | 1-4 | |
| | --- | | |
| Y | FR-A-2 264 077 (KELCO COMPANY) <br> * Claims 1,3; examples 17-19; page 5, line 37 - page 6, line 17 * | 1,3 | |
| | --- | | |
| D,Y | US-A-3 964 972 (J.T. PATTON) <br> * Whole document * | 1 | |
| | --- | | |
| A | FR-A-2 384 020 (TATE & LYLE LTD.) <br> * Claim 4; example 1; page 2, line 26 - page 3, line 6 * | 1 | |
| | --- | | **TECHNICAL FIELDS SEARCHED (Int. Cl. ³)** |
| P,A | EP-A-0 049 012 (SHELL INTERNATIONALE RESEARCH MAATSCHAPPIJ) <br> * Claims 1,6-8; page 4, lines 27-34; page 5, lines 25-30 * | 1,3,4 | C 12 P <br> C 12 N |
| | --- | | |
| P,A | EP-A-0 039 962 (SHELL INTERNATIONALE RESEARCH MAATSCHAPPIJ) <br> * Claims 1,5,7; examples 1,2; page 3, line 19 - page 5, line 7; in particular page 6, lines 22-29 * | 1 | |
| | ---     -/- | | |

The present search report has been drawn up for all claims

| Place of search | Date of completion of the search | Examiner |
|---|---|---|
| THE HAGUE | 10-02-1983 | DEKEIREL M.J. |

| DOCUMENTS CONSIDERED TO BE RELEVANT | | | Page 2 |
|---|---|---|---|
| Category | Citation of document with indication, where appropriate, of relevant passages | Relevant to claim | CLASSIFICATION OF THE APPLICATION (Int. Cl. 3) |
| E,X | GB-A-2 099 008 (INSTITUT FRANCAIS DU PETROLE) * Examples 5,6; page 2, lines 50-57; page 3, lines 22-30 * ----- | 1 | |
| | | | TECHNICAL FIELDS SEARCHED (Int. Cl. 3) |

The present search report has been drawn up for all claims

| Place of search THE HAGUE | Date of completion of the search 10-02-1983 | Examiner DEKEIREL M.J. |
|---|---|---|